# EUROPÄISCHE PATENTANMELDUNG

(11) **EP 1 992 371 A1**
(43) Veröffentlichungstag der Anmeldung: **19.11.2008**
(21) Anmeldenummer: 07108281.2
(22) Anmeldetag: 15.05.2007
(51) Int. Cl.: A61L 31/18, A61L 31/14, A61K 49/04, A61K 49/18

(54) **Bioresorbierbare röntgenopake Polymermaterialien und daraus hergestellte Occlussionsinstrumente**

(71) Anmelder: Occlutech GmbH, 07745 Jena (DE)
(72) Erfinder: Moszner, Robert, 07639, Bad Klosterlausnitz (DE); Schmidt, Kathrin, 07768, Kahla (DE); Moszner, Norbert, FL-9495 Triesen (LI); Schnabelrauch, Matthias, 07749, Jena (DE); Pautsch, Thomas, 07580, Paitzdorf (DE); Rode, Claudia, 07743, Jena (DE); Gottlöber, Ralf-Peter, 07422, Bad Blankenburg (DE)
(74) Vertreter: Krahbichler, Erik

(57) **Zusammenfassung**

Beschrieben werden bioresorbierbare und thermoplastisch verformbare Polymere mit oder ohne Formgedächtnis-Charakteristik, die in den Wiederholungseinheiten der Polymerketten röntgenopake Baugruppen enthalten und/oder mit bioresorbierbaren, röntgenopaken Nanopartikeln modifiziert sind. Die Polymere zeichnen sich weitgehend durch eine Kombination aus erforderlicher Festigkeit und hinreichender Deformierbarkeit, guter Bioresorbierbarkeit und ausreichender Sichtbarkeit im Röntgenlicht aus, und sind damit besonders zur Herstellung metallfreier Occlusionsinstrumente zum Verschluss von Defekten des Septums im Herzen geeignet, wobei deren Platzierung mit den üblichen röntgendiagnostischen Verfahren verfolgbar ist.

## Beschreibung

### Beschreibung

### Gebiet der Erfindung

Die vorliegende Erfindung betrifft bioresorbierbare, röntgenopake Polymere und deren Verwendung zur Herstellung von Occlusionsinstrumenten, die zum Verschluss von Defekten des Septums im Herzen eingesetzt werden und deren Platzierung mit röntgendiagnostischen Verfahren verfolgbar ist.

### Hintergrund der Erfindung

Für bestimmte medizinische Anwendungen, wie z.B. Stents, sind bestimmte röntgenopake bioresorbierbare Polymere bekannt. So werden röntgenopake, polymerbasierende Stents in der WO 2006/022754 (J. B. Kohn et al.) beschrieben. Es handelt sich um Polymere auf der Basis von halogenhaltigen (Br, I) Diphenolbausteinen, die über Dicarbonsäuren und/oder Poly(alkylenoxide) über Ester- oder Carbonatbindungen miteinander verbunden sind. Dabei werden die Diphenole durch Umsetzung von iodierten/nicht iodiertem Tyrosinethyl- oder -tert.-butylester, z.B. Tyrosinethylester (TE), mit iodierten/nicht iodiertem Desaminotyrosin, z.B. 3-(3,5-Diiod-4-hydroxyphenyl)-propionsäure (3,5-Diioddesaminotyrosin: I2DAT), vgl. Schema 1, hergestellt.

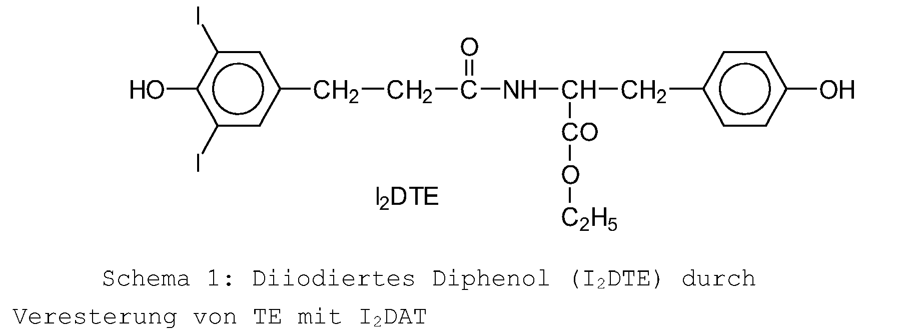

In der Patentanmeldung US 2006/0036316 A1 (J. Zeltinger et al.) werden analoge iodhaltige Diphenole über weitere Gruppen, wie Phosphat-, Phosphonat- oder Iminocarbonat-Gruppen, zu röntgenopaken, bioresorbierbaren Polymeren verknüpft.

Im Falle der voranstehenden Patentschriften erfolgt die Einbindung der röntgenopaken Gruppen direkt in die Polymerrückgratkette, was präparativ aufwendig ist und die Breite an Variationsmöglichkeiten deutlich einschränkt.

Demgegenüber werden in der Patentanmeldung US 2005/0036946 A1 (P. P. Chandrashekhar et al.) röntgenopake bioabbaubare Zusammensetzungen beschrieben, die auf synthetischen und natürlichen bioabbaubaren Polymeren beruhen, in denen iodhaltige Endgruppen eingebaut sind. Danach werden beispielsweise geeignet 2-fach terminierte (OH, NH₂) lineare Polymere, wie z.B. Poly(caprolacton), vgl. Schema 2, Poly(lactid) oder Polyether, oder n-fach terminier-te Pfropf-, Block- oder Sterncopolymere mit geeigneten iodhaltigen Derivaten, z.B. Triiodbenzoesäure oder Triiodphenol, endgruppenfunktionalisiert. Der Nachteil dieser röntgenopaken bioabbaubaren Polymeren besteht darin, dass damit Polymere nur mit einem geringen Iodgehalt und damit niedriger Röntgenopazität zugänglich sind.

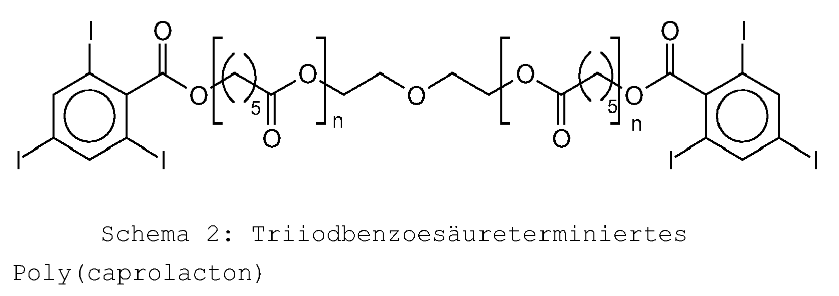

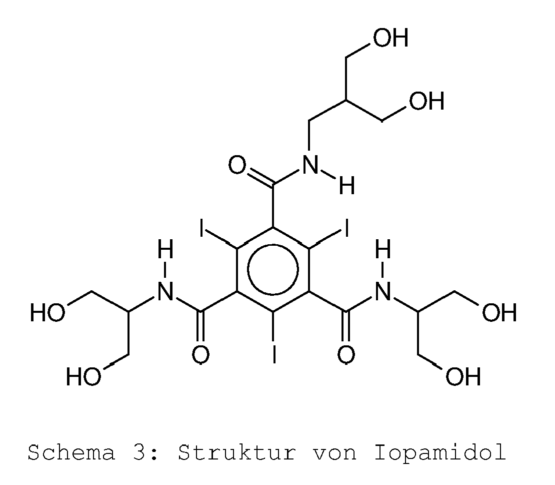

Chandrashekhar et al. beschreiben darüber hinaus in US 2005/0036946 A1 auch die Vernetzung von bioabbaubaren Biopolymeren, wie z.B. Albumin, Kollagen oder Chitosan mit geeigneten iodhaltigen Verbindungen, wie z.B. Iopamidol, siehe Schema 3, sowie die Verwendung der röntgenopaken bioabbaubaren Zusammensetzungen für die kontrollierte Freisetzung von Wirkstoffen. Jedoch ist auch bei dieser Synthesevariante von röntgenopaken bioabbaubaren Polymeren, aufgrund der meist hohen Molmasse der zugänglichen Biopolymeren, die erreichbare Röntgenopazität auf relativ geringe Werte beschränkt.

Ein bioresorbierbarer, röntgenopaker Marker für die Sichtbarmachung der Positionierung beim Einsatz von Endoprothesen ist in der Patentanmeldung US 2006/0004440 A1 (J.S. Stinson et al.) beschrieben. Dabei beruht die Polymermatrix der Marker auf bekannten bioresorbierbaren Polymeren, wie Poly(L-lactid) oder Poly(D-lactid), die langsamer oder Poly(glycolid) bzw. Poly(dioxanon), die schneller abbauen. Der Röntgenkontrast wird durch den Einbau von Metallpartikeln, z.B. der Elemente Ti, Zr, Pt oder Au, oder von organischen Verbindungen, die die Elemente Br, I, Ba oder Bi gebunden enthalten, realisiert.

Die Patentanmeldung WO 01/85214 A1 beschreibt röntgenopake Zusammensetzungen auf der Basis von Polymeren oder Monomeren, die nicht herauswaschbare röntgenopake Komponenten enthalten. Dabei wird der kovalente Einbau dieser röntgenopaken Komponenten beschrieben, wobei der Einbau durch Verknüpfung von bekannten röntgenopaken Verbindungen - auch verschiedenstartige Iodverbindungen - mit Monomer oder Polymer über geeignete funktionelle Gruppen, z.B. Isocyanat, Ester, Aldehyd oder Epoxid, erfolgt. Bei den verwendeten Polymeren werden bekannte synthetische oder natürliche Polymere aufgezählt, wobei jedoch bioabbaubare oder bioresorbierbare Polymere nicht erwähnt werden.

In der Patentanmeldung WO 02/089863 A1 werden Verfahren und Geräte zum Gefäßverschluss auf der Basis von metallfreien Werkstoffen, d.h. von einem oder mehreren bioabbaubaren Polymeren, beschrieben. Dabei können die Materialien bioaktive Wirkstoffe oder röntgenopake Additive enthalten. Dabei handelt es sich um bekannte Röntgenkontrastmittel, vor allem Metallpulver von Titan, Gold, Wolfram oder Bismut sowie Bariumsulfat oder auf Gandolonium basierende Verbindungen. Die Röntgenkontrastmittel sind dabei physikalisch in die verwendete Matrix an bioabbaubaren Polymer eingebettet. Dabei ist nachteilig, dass die physikalisch eingebundenen röntgenopaken Additive, die Verarbeitung und mechanischen Eigenschaften der metallfreien Werkstoffe deutlich beeinträchtigen. Darüber hinaus handelt es sich bei den beschriebenen röntgenopaken Additiven um nicht abbaubare, teilweise hinsichtlich ihrer Cytotoxizität bedenkliche, Substanzen. Deshalb verschlechtern die zugesetzten Metallpulver die Biokompatibilität der Werkstoffe.

Occlusionsinstrumente im Sinne der vorliegenden Anmeldung sind medizinische Produkte, die zum Verschluss von Defekten des Septums im Herzen, oder aber auch zum Verschluss eines Herzohres eingesetzt werden. Bei diesen Septumdefekten unterscheidet man vor allem die persistierenden Foramen ovale (PFO), Atriumseptumdefekte (ASD) und Ventrikelseptumdefekte (VSD). 2um Verschluss dieser Defekte verwendet man Occluder, die z.B. aus zwei Retensionsschirmen und einer dazwischen liegenden Taille aufgebaut sind. Die bisher eingesetzten Occluder werden aus einem Endlosdrahtgeflecht oder aus einem trichter- oder kugelförmigen Metalldrahtgeflechten hergestellt, wobei für die Drähte die Formgedächtnis-Legierung Nitinol verwendet wird. Nitinol ist eine Formgedächtnis-Legierung aus Nickel und Titan mit nur mäßiger Verformbarkeit und kann selbst oder durch entsprechende Korrosionsprodukte z.B. zu allergischen Abwehrreaktionen des Körpers führen. Demgegenüber zeigen Occluder auf der Basis von bioabbaubaren bzw. bioresorbierbaren Polymeren deutlich höhere Verformbarkeiten und eine verbesserte Biokompatibilität. Diese haben jedoch den gravierenden Nachteil, dass die Verfolgung der Platzierung der Occluder mit der üblichen Röntgendiagnostik nicht möglich ist.

Solche Occlusionsinstrumente auf der Basis von bioabbaubaren röntgenopaken Polymeren sind nach dem Stand der Technik nicht bekannt und es besteht ein Bedarf an solchen Occlusionsinstrumenten. Es besteht insbesondere ein Bedarf an Occlusionsinstrumenten bestehend aus vorteilhaften im Röntgenlicht sichtbaren, bioresorbierbaren und thermoplastisch verformbaren Polymeren.

### Zusammenfassung der Erfindung

Aufgabe der Erfindung ist es, die oben genannten Nachteile der herkömmlichen Vorrichtungen, zu überwinden, und/oder bioabbaubare röntgenopake Polymere bereitzustellen, die sich weitgehend durch eine Kombination aus erforderlicher Festigkeit und hinreichender Deformierbarkeit, guter Bioresorbierbarkeit und ausreichender Sichtbarkeit im Röntgenlicht auszeichnen und sich damit besonders zur Herstellung metallfreier Occlusionsinstrumente zum Verschluss von Defekten des Septums im Herzen eignen, wobei deren Platzierung mit den üblichen röntgendiagnostischen Verfahren verfolgbar ist.

Die oben genannte Aufgabe wird dadurch gelöst, dass die Vorrichtung der Erfindung die Merkmale des Anspruches 1 erhalten hat.

Diese Aufgabe wird somit erfindungsgemäß durch bioresorbierbare und thermoplastisch verformbare Polymere mit oder ohne Formgedächtnis-Charakteristik gelöst, die a) einerseits in den Wiederholungseinheiten der Polymerketten röntgenopake Gruppen enthalten und/oder b) andererseits mit bioresorbierbaren, röntgenopaken Nanopartikeln modifiziert sind.

Es wird ein vorteilhaftes bioabbaubares und/oder bioresorbierbares Material bereitgestellt, welches u.a.
o präparativ einfach zugänglich ist und eine Breite an Variationsmöglichkeiten bietet,
o sich durch thermische Verfahren oder aus Lösungen zu Fäden, Filmen oder Formkörpern verarbeiten lässt,
o eine erforderliche Festigkeit und hinreichende Deformierbarkeit für kollabierbare Occlusionsinstrumente aufweist,
o gute Bioresorbierbarkeit aufweist,
o ausreichende Sichtbarkeit im Röntgenlicht aufweist, und/oder
o sich besonders zur Herstellung metallfreier Occlusionsinstrumente zum Verschluss von Defekten des Septums im Herzen eignet, wobei deren Platzierung mit den üblichen röntgendiagnostischen Verfahren verfolgbar ist

### Kurzbeschreibung der Zeichnungen

Die Erfindung soll nachstehend anhand eines in der Zeichnung dargestellten beispielhaften Ausführungsbeispiels näher erläutert werden, wobei auf die beiliegenden Zeichnungen Bezug genommen wird.

Es zeigen:
Fig. 1: Beispiele für kommerzielle Triiodphenyl-Derivate;
Fig. 2: Beispiele für kommerzielle Triiodphenyl-Derivate;
Fig. 3: Beispiel für die Synthese eines Triiodphenyl-Seitengruppen-haltigen Diols;
Fig. 4: Diol **PE-GA-D(TIPh)** als Beispiel für eine Baugruppe mit Triiodphenyl-Seitengruppen entsprechend der untenstehenden Formel (I) (m = 2, (L = H, A = C(CH₂-)₄) ;
Fig. 5: Strukturen von bioabbaubaren Polyester;
Fig. 6: Strukturen von bioabbaubaren Polyanhydriden, Poly(aminosäure)n oder Polyamiden;
Fig. 7: Strukturen eines röntgenopaken, bioabbaubaren Poly(milchsäure)-diols;
Fig. 8: Synthesemöglichkeiten von kovalenten, röntgenopaken, bioabbaubaren Polymernetzwerken;
Fig. 9: Beispiele für bekannte radikalisch polymerisierbare Triiod-Monomere;
Fig. 10: Beispiele für ein bioabbaubaren Polyorthoester und Polyphosphazen;
Fig. 11 und Fig. 12: mittels einer Flechtmaschine ausgebildete Geflechte aus röntgenopaken, bioabbaubaren Polymerfilamenten; und
Fig. 13: beispielhafte Formen für Occlusionsinstrumente, im expandierten Zustand.

### Ausführliche Beschreibung der Erfindung

Der Fachmann wird nachvollziehen können, dass offensichtliche Modifikationen der beschriebenen Ausführungsformen der Erfindung möglich sind, ohne vom Umfang der beigefügten Patentansprüche abzuweichen.

Wie oben erwähnt, wird die durch die Erfindung zu lösende Aufgabe gemäß Ausführungsformen der Erfindung durch bioresorbierbare und thermoplastisch verformbare Polymere mit oder ohne Formgedächtnis-Charakteristik gelöst, die
a) einerseits in den Wiederholungseinheiten der Polymerketten röntgenopake Gruppen enthalten und/oder
b) andererseits mit bioresorbierbaren, röntgenopaken Nanopartikeln modifiziert sind. Dies wird nachstehend näher erläutert.

### a) Röntgenopake Gruppen

Gemäß einer Ausführungsform der Erfindung sind die im Röntgenlicht sichtbaren, bioresorbierbaren und thermoplastisch verformbaren Polymeren dadurch charakterisiert, dass sie folgende röntgenopake Baugruppen mit Triiodphenyl-Seitengruppen entsprechend der Formel (I) enthalten: 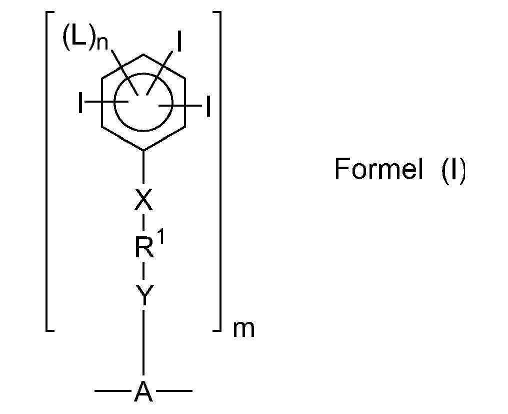 wobei
L H oder eine wasserlöslichkeitsvermittelnde Carboxylat-, Ammonium-, Phosphat-, Phosphonat-, Sulfat- oder Sulfonat-Gruppe oder ein Oligoethylenoxid- oder Acetylamino-Rest darstellt,
n zwischen 0, 1 oder 2 variieren kann,
Y, X entfällt oder Verbindungsgruppen, wie Ether-, Carbonsäureester- oder Carbonsäureamid- oder Urethan-Gruppen darstellen,
R¹ entfällt oder ein 2-wertiger linearer, verzweigter oder cyclischer organischer Rest ist, der aus 1 bis 15 Kohlenstoffatomen besteht,
A ein m+2-wertiger linearer, verzweigter oder cyclischer organischer Rest ist, der 1 bis 30 Kohlenstoffatome enthält und
m zwischen 1 und 4 variieren kann und die Substitution des Phenylrestes durch die Iod-Atome in freie o- m- oder p-Positionen erfolgen kann.
Dabei ist in einer Ausführungsform besonders bevorzugt, wenn
L H, eine wasserlöslichkeitsvermittelnde Carboxylat-, Ammonium-, Phosphat-, Sulfat-Gruppe oder ein Oligoethylenoxid- oder Acetylamino-Rest darstellt,
n zwischen 0, 1 oder 2 variiert,
Y, X entfällt oder Verbindungsgruppen, wie Carbonsäureester- oder Urethan-Gruppen darstellen,
R¹ entfällt oder ein 2-wertiger linearer, verzweigter oder cyclischer organischer Rest ist, der aus 1 bis 10 Kohlenstoffatomen besteht,
A ein m+2-wertiger linearer, verzweigter oder cyclischer organischer Rest ist, der 1 bis 20 Kohlenstoffatome enthält und
m zwischen 1 und 2 variiert und die Substitution des Phenylrestes durch die Iod-Atome in freie o- m- oder p-Positionen erfolgt.

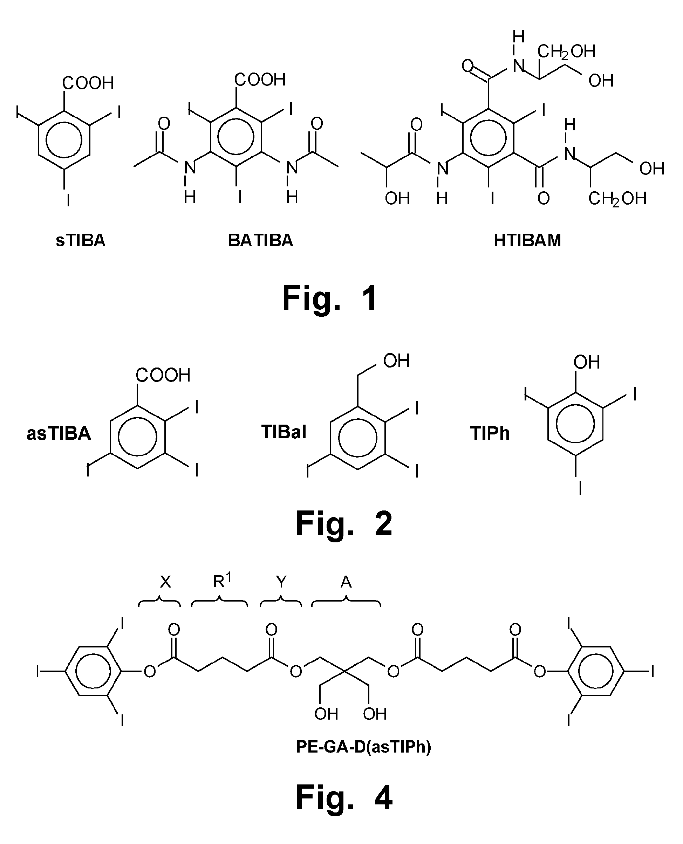

Weiterhin sind Baugruppen mit Triiodphenyl-Seitengruppen besonders geeignet, die sich von kommerziellen Triiodphenyl-Derivaten ableiten, wie z.B. 2,4,6-Triiodbenzoesäure (**sTIBA**), 3,5-Bis(acetamido)-2,4,6-triiodbenzoesäure (**BATIBA**, Diatrizoic acid) oder 5-(α-Hydroxypropionylamino-2,4,6-triiodisophthalsäure-di(1,3-hydroxyisopropylamid) (**HTIBAM**, Iopamidol), wie in Fig. 1 dargestellt.

Außerdem kommen als Synthesebausteine folgende kommerzielle Triiodverbindungen in Frage: 2,3,5-Triiodbenzoesäure **(asTIBA),** 2,3,5-Triiodbenzylalkohol **(TIBa1),** oder 2,4,6-Triiodphenol **(TIPh),** siehe Fig. 2. Mit diesen Triiodphenyl-Derivaten lassen sich durch Reaktion mit geeigneten multifunktionellen organischen Verbindungen, gegebenenfalls unter Berücksichtigung der Schutzgruppen-Technik geeignete röntgenopake Baugruppen mit Triiodphenyl-Seitengruppen entsprechend der Formel (I) herstellen.

Dieses Herstellungsverfahren wird nachfolgend an einem Beispiel erläutert.

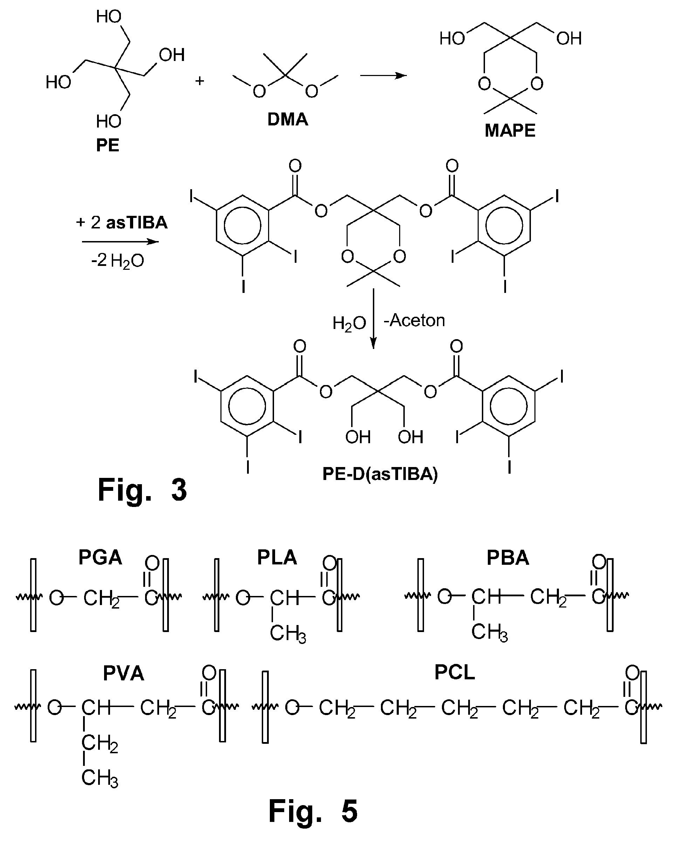

So kann Pentaerythrit (**PE**) in einer 1. Stufe durch Ketalisierung mit Dimethoxyaceton (**DMA**) zu einem Monoaceton-Pentaerythrit (**MAPE**) umgesetzt werden. Anschließend lassen sich die beiden freien OH-Gruppen in einer 2. Stufe mit 2,3,5-Triiodbenzoesäure (**asTIBA**) verestern. Schließlich wird in einer 3. Stufe die Aceton-Schutzgruppe sauer hydrolytisch wieder abgespalten, wodurch sich das zwei Triiodphenyl-Seitengruppen enthaltene Diol **PE-D(asTIBA)** bildet. Dies ist in Fig. 3 illustriert.

Dabei kann die Synthese alternativ auch so erfolgen, dass **MAPE** zum Einbau eines Spacers zunächst mit Glutarsäureanhydrid (**GA**) verestert wird, dann durch Umsetzung mit 2,4,6-Triiodphenol (**TIPh**) der Einbau der beiden Triiodphenyl-Seitengruppen erfolgt und schließlich in der letzten Stufe die Aceton-Schutzgruppe abgespalten wird, wodurch sich das wiederum zwei Triiodphenyl-Seitengruppen enthaltene Diol **PE-GA-D(TIPh)** bildet, siehe Fig. 4.

Gemäß Ausführungsformen der Erfindung können neben Pentaerythrit in analoger Weise andere multifunktionelle organische Verbindungen mit mindestens drei gleichen oder unterschiedlichen funktionellen Gruppen zur Herstellung der röntgenopaken Baugruppen verwendet werden. Die funktionellen Gruppen können z. B. Hydroxyl-, Amino-, Thiol- oder Carboxylgruppen sein. Besonders geeignet erscheinen dabei hydroxylgruppenhaltige Verbindungen mit mehr als drei Hydroxylgruppen pro Molekül. Diese Verbindungen können neben Hydroxylgruppen weitere funktionelle Gruppen enthalten. Hydroxylgruppenhaltige Verbindungen, die zur Herstellung der erfindungsgemäßen röntgenopaken Bausteine geeignet sind, umfassen neben Pentaerythrit beispielsweise Erythrit, Xylit, Sorbit, Inosit, Methylglucosid oder Chinasäure. Entsprechend der Art und Anzahl der funktionellen Gruppen in diesen multifunktionellen Verbindungen lassen sich unter Nutzung von geeigneten, nach dem Stand der Technik bekannten Schutzgruppentechniken erfindungsgemäße röntgenopake Baugruppen synthetisieren.

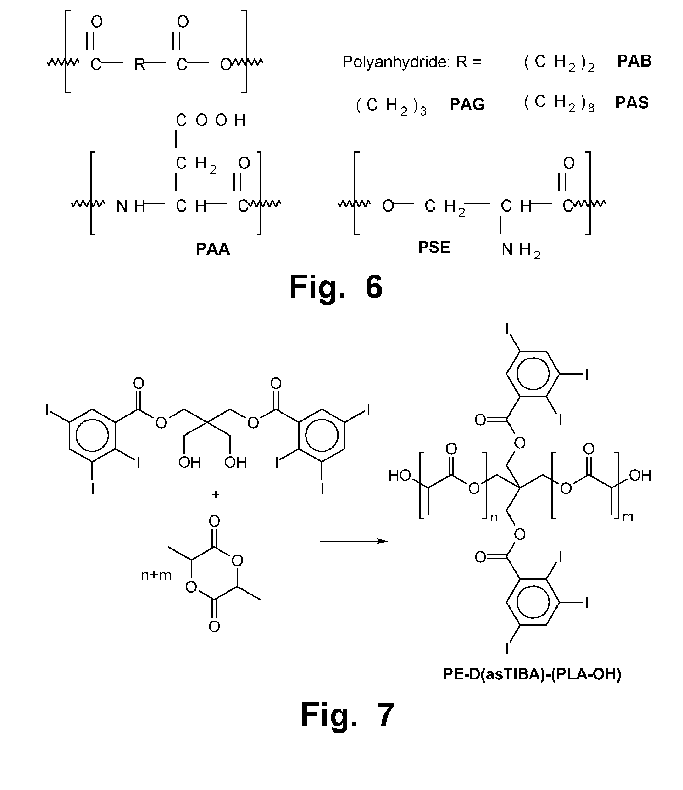

Die röntgenopaken Baugruppen der Formel (I) lassen sich in bioresorbierbare Polymere durch Copolymerisation, Cokondensation bzw. Polyaddition einbauen. Dabei können folgende bekannte bioabbaubare synthetische Polymerklassen (vgl. J. M. Mayer, D. L. Kaplan, Trends Polym. Sci. 2(1994) 227) zur Anwendung kommen:
- Polyester, wie Poly(milchsäure) **PLA**, Poly(glycolsäure) **PGA**, Poly(3-hydroxybuttersäure) **PBA**, Poly(4-hydroxyvaleriansäure) **PVA** oder Poly(ε-caprolaton) **PCL** bzw. entsprechende Copolymere, siehe Fig. 5;
- Polyanhydride, die aus Dicarbonsäuren wie z.B. Glutar- **PAG**, Bernstein- **PAB** oder Sebacinsäure **PAS** gebildet werden siehe Fig. 6; oder
- Poly(aminosäure)n oder Polyamide wie z.B. Poly(serinester) **PSE** oder Poly(asparaginsäure) **PAA** siehe Fig. 6.

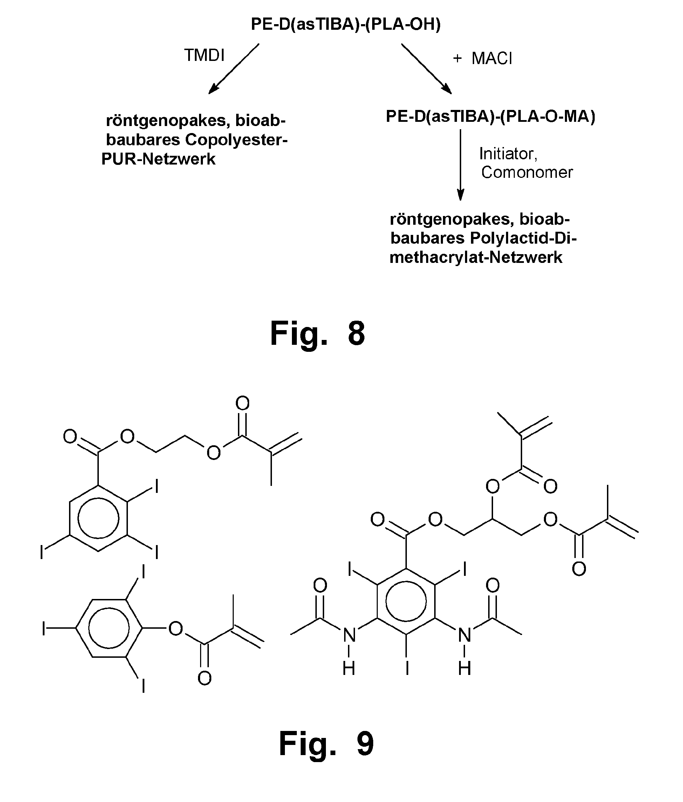

So können die röntgenopaken Baugruppen der Formel (I) wie z.B. das Diol **PE-D (asTIBA)** als Starteralkohol für die Ringöffnungspolymerisation z.B. von Lactid eingesetzt werden. Die so gebildeten OH-terminierten bioabbaubaren, röntgenopaken Polymeren **PE-D(asTIBA)-(PLA-OH),** siehe Fig. 7, lassen sich dann mit kommerziellen Diisocyanaten, wie z.B. Trimethylhexamethylendiisocyanat (TMDI) unter Bildung eines bioabbaubaren Polyurethannetzwerkes vernetzen, wie in Fig. 8 dargestellt ist.

Weiterhin lassen sich die OH-terminierten, bioabbaubaren, röntgenopaken Polymeren **PE-D(asTIBA)-(PLA-OH)** durch Umsetzung z.B. mit Methacrylsäurechlorid (MAC1) in radikalisch polymerisierbare, bioabbaubare, röntgenopake Telechele umwandeln. Diese Telechele können dann in Gegenwart eines radikalischen Initiators und gegebenenfalls von weiteren radikalisch polymerisierbaren Comonomeren copolymerisiert werden, wobei dann ein bioabbaubares, röntgenopakes, kovalentes Polymernetzwerk gebildet wird. In diesem Zusammenhang ist es vorteilhaft, als Comonomere bekannte, kommerziell gut zugängliche und als bioverträglich bekannte, röntgenopake Mono- oder Dimethacrylate einzusetzen, siehe Fig. 9, und vgl. N. Moszner, u. Salz, A. M. Klester, V. Rheinberger, Angew. Makromol. Chem. 224 (1995) 115, K. W. M. Davy, M. R. Anseau, M. Odlyha, G. M. Foster, Polym. Intern. 43 (1997) 143. Ein Vorteil besteht hierbei darin, dass man auf Comonomere mit bekannter Reaktivität und Röntgenopazität zurückgreifen kann.

Außerdem kann eine Vernetzung auch nach einer Fadenbildung der Komponenten bzw. nach der Herstellung von Formteilen, wie z.B. Occlusionsinstrumenten, erfolgen. Besonders geeignet ist dabei eine strahlungsinduzierte Vernetzung unter Verwendung von energiereicher Strahlung, wie z.B. γ-Strahlung. In diesem Zusammenhang kann eine weitere Verbesserung der mechanischen Eigenschaften, wie der Reißfestigkeit oder des Elastizitätsmoduls, durch gezielte Auswahl der Strahlungsbedingungen, z.B. der Strahlungsdosis, erreicht werden.

Für die Bioresorbierbarkeit der röntgenopaken, thermoplastisch verformbaren Polymeren ist von Vorteil, dass die röntgenopaken Baugruppen mit Triiodphenyl-Seitengruppen entsprechend der Formel (I), die beim Bioabbau freigesetzt werden, eine hinreichende Löslichkeit in Wasser besitzen. Ein Vorteil von wasserlöslichen Triiodphenyl-Seitengruppen besteht darin, dass sie zu besonders gut bioresorbierbaren Polymeren führen. Dies kann durch den Einbau von wasserlöslichkeitsvermittelnden Carboxylat-, Ammonium-, Phosphat-, Phosphonat-, Sulfat- oder Sulfonat-Gruppen oder von Oligoethylenoxid- oder Acetylamino-Resten erreicht werden.

Zur Modifizierung und Einstellung der mechanischen, thermischen und/oder spezifischen Applikationseigenschaften können den röntgenopaken, thermoplastisch verformbaren Polymeren vor oder während der Formgebung Additive zugesetzt werden. Solche Additive sind beispielsweise Weichmacher, polymere oder niedermolekulare organische Füllstoffe, Farbstoffe, die Biodegradation beeinflussende Substanzen oder zusätzlich die Röntgenopazität verstärkende anorganische oder organische Verbindungen.

### b) Modifikation mit röntgenopaken Nanopartikeln

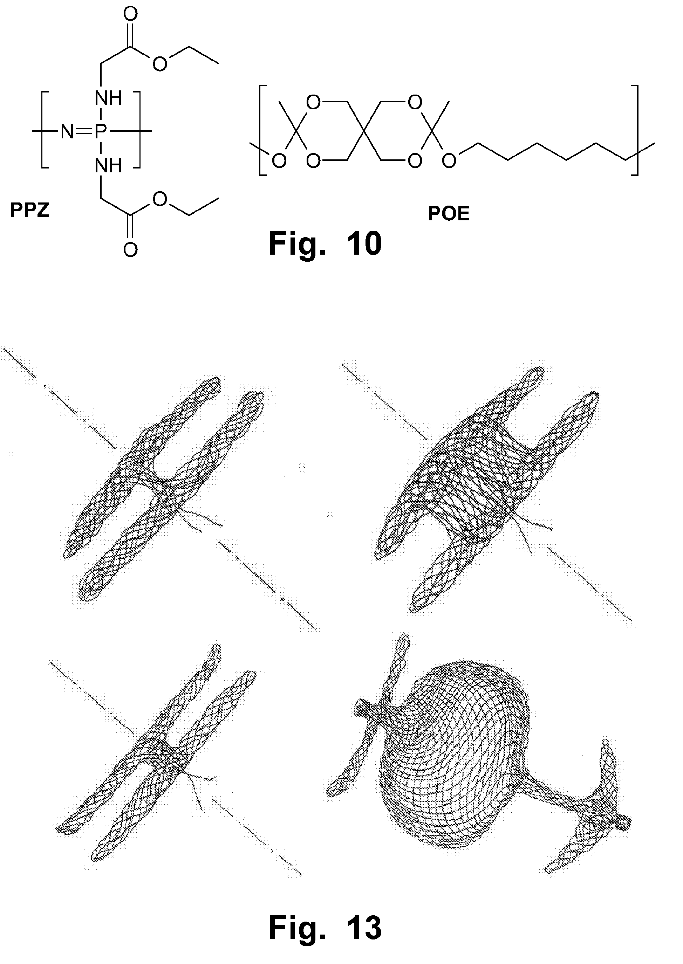

Die erfindungsgemäße Modifizierung der bioresorbierbaren und thermoplastisch verformbaren Polymeren mit oder ohne Formgedächtnis-Charakteristik erfolgt mit bioresorbierbaren, röntgenopaken Nanopartikeln. Diese Nanopartikel lassen sich durch Nanoverkapselung von bioresorbierbaren, röntgenopaken Verbindungen herstellen. Die Mikro- oder Nanoverkapselung von feindispersen flüssigen oder festen Komponenten durch Umhüllung mit filmbildenden Polymeren ist eine bekannte Technologie, die z.B. zum Schutz von weniger stabilen Komponenten vor Umgebungseinflüssen, zur Verringerung des Geruchs von übelriechenden Komponenten oder zur Herstellung von Medikamenten mit kontrollierter Wirkstofffreisetzung, eingesetzt wird, vgl. C. A. Finch, in: Ullmann's Encyclopedia of Industrial Chemistry, 4. Ed., Vol. A16, VCH Verlagsgesellschaft, Weinheim etc. 1993, 575; C. Thies, in: Encylopedia of Polymer Science and Engineering, Vol. 9, John Wiley & Sons, New York etc. 1988, 724. Je nach Herstellungsverfahren lassen sich dabei die für die erfindungsgemäße Modifizierung der bioresorbierbaren und thermoplastisch verformbaren Polymeren benötigten Nanokapseln in einer Größe von ca. 50 bis 1000 nm herstellen. Die einzelnen Herstellungsverfahren von Nanokapseln unterscheiden sich danach, ob Monomere oder Polymere als Ausgangsstoffe für die Wandbildung verwendet werden und ob die Wandbildner in einer der Phasen (Kern- oder kontinuierliche Phase) oder in beiden vorgelegt werden. Dabei ist es meist nicht notwendig, dass perfekt sphärische Kapsel gebildet werden. Die besonders kleinen Nanokapseln lassen sich über sog. Miniemulsionen herstellen, vgl. N. Bechthold, F. Tiarks, M. Willert, K. Landfester, M. Antonietti, Macromol. Symp. 2000, 151, 549. Für die erfindungsgemäßen bioresorbierbaren, röntgenopaken Nanopartikel ist es vorteilhaft, ein wasserlösliches oder bioabbaubares Polymer als Hüllmaterial einzusetzen. Der Vorteil der Verwendung von wasserlöslichen oder bioabbaubaren Hüllpolymer besteht darin, dass sie zu besonders gut bioresorbieren Nanopartikel führen. Als wasserlösliche Polymere kommen beispielsweise kommerziell verfügbare Stärke oder Cellulosederivate, wie z.B. Natriumalginat oder Carboxymethylcellulose, sowie Pullulan, Polyvinylalkohol oder Gelatine in Frage. Als nichtwasserlösliche, bioabbaubare Polymere sind erfindungsgemäß die schon voranstehend genannten Polyester auf der Basis von α-Hydroxycarbonsäuren, wie Milchsäure oder Glycolsäure, sowie deren Copolymere, Polyanhydride und Poly(α-aminosäure)n besonders geeignet. Außerdem lassen sich auch Polyorthoester (**POE**) oder Polyphosphazene (**PPZ**) verwenden, vgl. Fig. 10.

Als Kernmaterial für die bioresorbierbaren, röntgenopaken Nanopartikel können geeignete Derivate der kommerziellen Triiodphenyl-Verbindungen, wie z.B. 2,4,6-Triiodbenzoesäure **(sTIBA),** 3,5-Bis(acetamido)-2,4,6-triiodbenzoesäure **(BATIBA,** Diatrizoic acid), 5-(α-Hydroxypropionylamino-2,4,6-triiodisophthalsäure-di(1,3-hydroxy-isopropylamid) **(HTIBAM,** Iopamidol) (Fig. 1), 2,3,5-Triiodbenzoesäure **(asTIBA),** 2,3,5-Triiodbenzylalkohol **(TIBa1)** oder 2,4,6-Triiodphenol **(TIPh)** (Fig. 2) verwendet werden.

Durch die gezielte Auswahl bestimmter Polymere lassen sich die Geschwindigkeit der Bioabbaubarkeit bzw. Bioresorption, die Quellbarkeit, die Stabilität und mechanischen Eigenschaften der Polymernanokapseln gezielt einstellen.

Durch Schmelz- oder Lösungsspinnen der erfindungsgemäßen bioresorbierbaren und thermoplastisch verformbaren Polymeren mit oder ohne Formgedächtnis-Charakteristik, die einerseits in den Wiederholungseinheiten der Polymerketten röntgenopake Triiodphenyl-Seitengruppen entsprechend der oben stehenden Formel (I) enthalten und/oder andererseits die mit bioresorbierbaren, röntgenopaken Nanopartikeln modifiziert sind, lassen sich Fäden herstellen. Diese Fäden können sowohl aus einem einzigen Faden, einem so genannten Monofil, als auch aus mehreren Einzelfilamenten, welche dann ein so genanntes Multifilament (auch Multifilamentgarn genannt) bilden, bestehen, wobei Monofile für gewöhnlich dicker als Einzelfilamente der Multifilamentgarne sind. Aus der Schmelze geformte Fäden werden nach dem Spinnen abgekühlt und die dabei stattfindende Wärmeübertragung durch gekühlte Medien verbessert. Die Festigkeit der erhaltenen Fäden wird bei Bedarf durch Recken gesteigert, weitere Verfahren zur Steigerung der Festigkeit sind thermische Behandlung, Vernetzung oder auch Kombinationen dieser Verfahren. Das Recken lässt sich durch Erwärmung der Fäden unterstützen, wobei die Erwärmung durch Luft oder andere Gase, zum Beispiel Stickstoff, Flüssigkeiten oder Strahlung (Mikrowellen, IR-Strahlung) erfolgen kann.

Der Zusammenhalt des das Multifilamentgarn bildenden Verbands aus Einzelfilamenten kann durch Aufbringen von Drehungen und/oder durch Verwirbeln verbessert werden. Neben diesen Verfahren der Verbesserung des Zusammenhalts ist auch die Nutzung adhäsiv wirkender Substanzen möglich. Auf Basis der Fäden aus bioresorbierbaren, röntgenopaken Polymeren können dann entsprechende Geflechte oder auch andere geeignete textile Flächengebilde für Occlusionsinstrumente hergestellt werden.

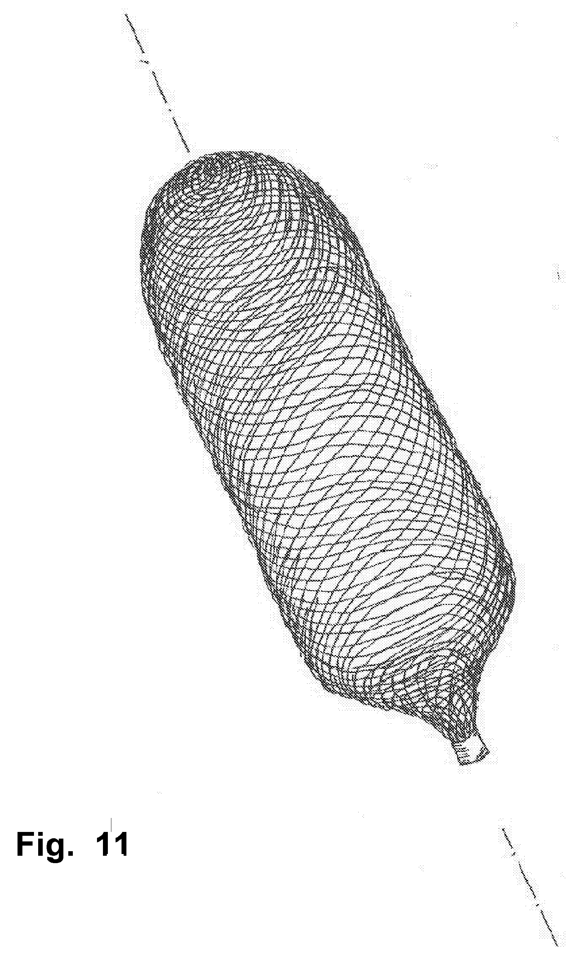

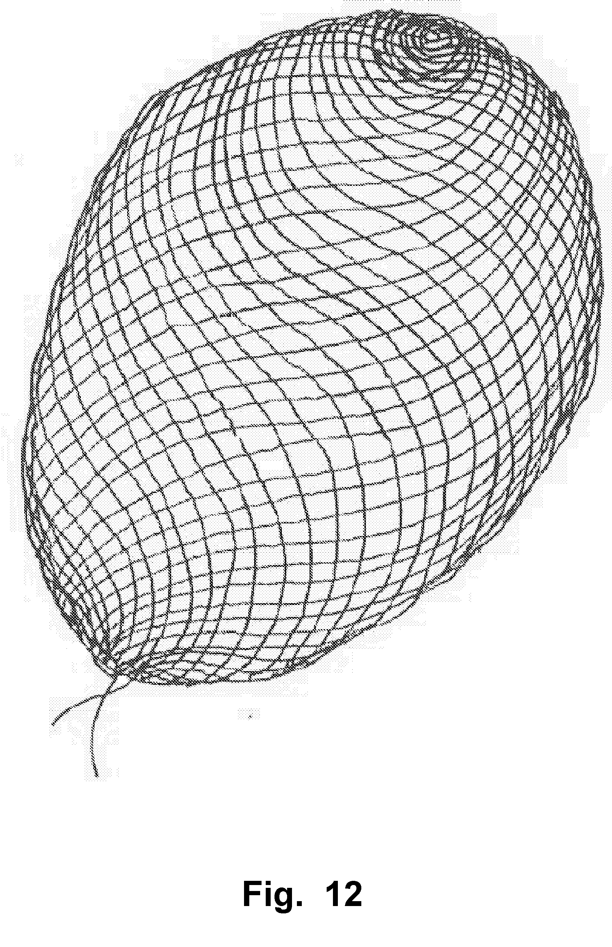

Dabei werden zunächst flexible Endlosgeflechte bzw. flexible trichter- oder kugel/birnen/tropfenförmiges Geflecht mittels einer Flechtmaschine ausgebildet, siehe Fig. 11 und Fig. 12. Insbesondere können zur Geflechtherstellung Multi- oder Monofilamente verwendet werden. Durch bekannte Flechttechnologie lassen sich die trichter- oder kugel/birnen/tropfenförmigen Geflechte in einem Ende bündeln und fassen, siehe DE 10338702; DE 102006013770. Bei der Herstellung von Endlosgeflechten wird jeweils beide Enden des Geflechtes gebündelt und gefasst. Das geflochtene Gewebe wird dann durch einen Wärmebehandlungsschritt in die gewünschte Form gebracht, wobei die Formgebung durch die konstruktive Gestaltung der Vorrichtung gegeben ist. Hierbei können insbesondere induktive oder thermische Wärmebehandlungs-verfahren verwendet werden. Dauer und Temperatur der Wärmebehandlung wird so gewählt, dass das geflochtene Gewebe seine Verformung beibehält. Nach der Wärmebehandlung wird die formgebende Vorrichtung entfernt. Das Geflecht behält dabei seine Verformung bei. Das so behandelte Flechtgewebe entspricht der vorab festgelegten (expandierten) Form des medizinischen Occlusionsinstruments, dass im zusammengefalteten Zustand mittels eines Kathetersystems implantiert werden kann. Einige beispielhafte Formen solcher Occlusionsinstrumente sind in Fig. 13 dargestellt.

### Beispiele

Die Erfindung wird im folgenden anhand von Beispielen näher erläutert.

### Beispiel 1: Synthese von PE-D(asTIBA)

50 g (0,1 mol) Triiodbenzoesäure , 8,81 g (0,05 mol) Monoaceton-Pentaerythrit und 12,22 g (0,1 mol) Dimethylaminopyridin (DMAP) wurden in trockenem Methylenchlorid gelöst. Unter Eiskühlung wurden 20,63 g (0,1 mol) Dicyclohexylcarbodiimid (DCC) spatelweise zugegeben und gerührt. Innerhalb weniger Minuten fiel ein weißer Niederschlag von Dicyclohexlharnstoff (DCH) aus. Es wurden weitere 15 Stunden bei Raumtemperatur gerührt und der Feststoff abfiltriert. Dieser wurde mehrmals mit THF gewaschen. Die vereinigten Lösungen wurden eingeengt. Dabei wurde ein zähflüssiges braunes Öl erhalten, welches mit Essigsäureethylester versetzt wurde. Dabei fiel ein weißer Niederschlag an, welcher abfiltriert und anschließend durch Säulenchromatographie weiter gereinigt wurde (Kieselgel 60, Laufmittel Essigsäureethylester/Heptan 1:1). Ausbeute: 65%. Fp: 142 °C.

Elementaranalytische Iodid-Bestimmung: I: 66,51 % (ber.: 66,80 %).

Zur Abspaltung der Isopropyliden-Schutzgruppe wurde das erhaltene Zwischenprodukt mit einem Überschuss an 1N HCl in THF versetzt und 24 Stunden bei Raum-temperatur gerührt. Zur Aufarbeitung wurde das THF abdestilliert und das Produkt in Methylenchlorid aufgenommen. Die organische Phase wurde zweimal mit je 100 ml gesättigter NaHCO3-Lösung und konzentriertem Salzwasser ausgeschüttelt und anschließend über Natriumsulfat getrocknet. Nach dem Filtrieren und Einengen der Lösung wurde das Produktgemisch säulenchromatografisch aufgearbeitet (Kieselgel 60, Laufmittel Essigsäureethylester/Heptan 1:1). Man erhielt das Produkt in 60%-iger Ausbeute in Form eines weißen Pulvers (Fp: 146 °C). Elementaranalytische Iodid-Bestimmung: I: 70,99 % (ber.: 69,24 %).

¹H-NMR (ppm, THF-D₈) : 3,57 und 3,59 (d, 4H, CH₂); 3,85-3,87 (t, 2H, OH); 4,31 (s, 4H, CH₂); 7,65 (s, 2H, CH) und 8,26 (s, 2H, CH.

IR (cm⁻¹, KBr): 3295 (OH-Valenzschwingung); 3067 (C-H-Valenzschwingung); 1733 und 1709 (C=O-Valenzschwin-gung); 1178 (C-O-Valenzschwingung der COOH-Gruppe); 1056 (C-O-Valenzschwingung der CH₂OH-Gruppe).

### Beispiel 2: Synthese von PE-D(asTIBA)-(PLA-O-PAS)

Ein Gemisch aus 1,0 g (0,9 mmol) PE-D(asTIBA), 3,93 g (27,3 mmol) Lactid und 11 mg Zinn(II)-2-ethylhexanoat wurden unter Feuchtigkeitsausschluss 3 h bei 150°C gerührt. Nach Abkühlen des Gemisches auf Raumtemperatur wurde 50 ml getrocknetes Methylenchlorid zugegeben und bis zum vollständigen Lösen gerührt. Anschließend wurden 5 ml wasserfreies Triethylamin zugegeben und dann wurden unter Eiskühlung, Feuchtigkeitsausschluss und Rühren 0,22 g (0,9 mmol) Sebacinsäure-dichlorid zugetropft. Das Reaktionsgemisch wurde unter Rühren auf Raumtemperatur erwärmt und weitere 15 h gerührt. Nach der Zugabe von 50 ml Methylenchlorid wurde das ausgefallene Triethylammoniumhydrochlorid abfiltriert und der Rückstand mit wenig Methylenchlorid gewaschen. Anschließend wurde das Reaktionsgemisch nacheinander zweimal mit je 120 ml 1N Salzsäure, zweimal mit je 120 ml gesättigter NaHCO3-Lösung und dreimal mit je 200 ml Wasser ausgeschüttelt. Der Extrakt wur-de über wasserfreiem Natriumsulfat getrocknet, filtriert und im Vakuum eingeengt. Man erhält einen gelben Feststoff in einer Ausbeute von 80%. Die

Molmassen-bestimmung mittels GPC ergab: Mn: 29.600 g/mol; Mw: 43.500 g/mol; D: 1,47 (UV-Detektion). Durch Lösungs- öder Schmelzspinnen kann das erhaltene Polymer zu Fäden verarbeitet werden. Die elementaranalytische Iodid-Bestimmung ergab: I: 13,77 % (ber.: 13,60 %). Dieser IodGehalt führt bei einer Prüfkörperdicke von ca. 0.77 mm zu einer Röntgenopazität von 95 % Aluminium (Al). Damit liegt die Röntgenopazität der Polymeren im Bereich von Occludern aus Nitinol, die eine über den gesamten Occluder unterschiedliche Röntgenopazität zeigen, d.h. die Röntgenopazität beträgt im Inneren des Occluders ca. 60 % Al, in der Mitte ca. 80 % Al und im Randbereich ca. 168 % Al.

### Beispiel 3: Herstellungsvarianten von Mono- oder Multifilamenten

**a)** Das Polymer bzw. Polymercompound wurde mit einem Extruder aufgeschmolzen, wobei die Temperatur der Schmelze um 5 bis 15 °C über dem mittels DSC oder Heiztischmikroskop ermittelten Schmelzbereich lag. Die Schmelze wurde mittels Zahnradpumpe durch die Bohrung einer Spinndüse für Monofilamente gepresst, im Wasserbad abgekühlt und mit einer Geschwindigkeit von 10 bis 75 m/min abgezogen. Dieses Monofil wurde im Wasserbad bei Temperaturen von 30 bis 80°C bis zu 20-fach gereckt, wobei ein gerecktes Monofilament mit einem Durchmesser von ca. 0,05 mm und Festigkeiten von mehr als 40 cN/tex erhalten wurde.
**b)** Das Polymere bzw. Polymercompound wurde mit einem Extruder aufgeschmolzen, wobei die Temperatur der Schmelze um 5 bis 15 °C über dem mittels DSC oder Heiztischmikroskop ermittelten Schmelzbereich lag. Die Schmelze wurde mittels Zahnradpumpe durch die Bohrungen einer Spinndüse für Multifilamentgarne gepresst, im Wasserbad abgekühlt und mit einer Geschwindigkeit von 10 bis 75 m/min abgezogen. Dieses Multifilamentgarn wurde im Wasserbad bei Temperaturen von 30 bis 80 °C bis zu 10-fach gereckt, wobei ein gerecktes Multifilamentgarn mit Durchmessern der Einzelfilamente von 25 µm erhalten wurde.
**c)** Das Polymere bzw. Polymercompound wurde mit einem Extruder aufgeschmolzen, wobei die Temperatur der Schmelze um 5 bis 15 °C über dem mittels DSC oder Heiztischmikroskop ermittelten Schmelzbereich lag. Die Schmelze wurde mittels Zahnradpumpe durch die Bohrungen einer Spinndüse für Multifilamentgarne gepresst, in strömenden Gasen abgekühlt und mit einer Geschwindigkeit von 20 bis 750 m/min abgezogen. Dieses Multifilamentgarn wurde im Wasserbad bei Temperaturen von 30 bis 80 °C bis zu 8-fach gereckt, wobei ein gerecktes Multifilamentgarn mit Durchmessern der Einzelfilamente von 15 µm und Festigkeiten von mehr als 40 cN/tex (>290 MPa) erhalten wurde.
**d)** Aus dem Polymeren bzw. Polymercompound wurde mit Methyletherketon als Lösungsmittel eine 8%-ige Lösung hergestellt. Die Lösung wurde mittels Zahnradpumpe durch die Bohrungen einer Spinndüse für Multifilamentgarne gepresst, im Fällbad ausgefällt und mit einer Geschwindigkeit von 10 bis 50 m/min abgezogen. Dieses Multifilamentgarn wurde im Wasserbad bei Temperaturen von 30 bis 80 °C bis zu 10-fach gereckt, wobei ein gerecktes Multifilamentgarn mit Durchmessern der Einzelfilamente von 15 µm und Festigkeiten von mehr als 22 cN/tex erhalten wurde.
**e)** Das Polymere bzw. Polymercompound wurde mit einer Kolbenspinnapparatur aufgeschmolzen, wobei die Temperatur der Schmelze um 5 bis 15 °C über dem mittels DSC oder Heiztischmikroskop ermittelten Schmelzbereich lag. Die Schmelze wurde mittels Kolben durch die Bohrung einer Spinndüse für Monofilamente gepresst, im Wasserbad abgekühlt und mit einer Geschwindigkeit von 5 bis 75 m/min abgezogen. Dieses Monofil wurde im Wasserbad bei Temperaturen von 30 bis 80 °C bis zu 20-fach gereckt, wobei ein gerecktes Monofilament mit einem Durchmesser von 0,04 mm und Festigkeiten von mehr als 35 cN/tex erhalten wurde.
**f)** Aus dem Polymeren bzw. Polymercompound wurde mit Aceton als Lösungsmittel eine 6%-ige Lösung hergestellt. Die Lösung wurde mittels Kolben einer Kolbenspinnaparatur durch die Bohrungen einer Spinndüse für Multifilamentgarne gepresst, im Fällbad ausgefällt und mit einer Geschwindigkeit von 10 bis 50 m/min abgezogen. Dieses Multifilamentgarn wurde im Wasserbad bei Temperaturen von 30 bis 80 °C bis zu 10-fach gereckt, wobei ein gerecktes Multifilamentgarn mit Durchmessern der Einzelfilamente von 12 µm und Festigkeiten von mehr als 25 cN/tex erhalten wurde.
**g)** Aus dem Polymeren bzw. Polymercompound wurde mit den gleichen Substanzen wie in Beispiel 3f) beschrieben, eine 4,37%-ige Lösung hergestellt. Die Lösung wurde mittels Zahnradpumpe durch die Bohrungen einer Spinndüse für Multifilamentgarne gepresst, im Fällbad ausgefällt und mit einer Geschwindigkeit von 5 bis 105 m/min abgezogen. Dieses Multifilamentgarn wurde im Wasserbad bei Temperaturen von 30 bis 80 °C bis zu 10-fach gereckt, wobei ein gerecktes Multifilamentgarn mit Durchmessern der Einzelfilamente von 15 µm und Festigkeiten von mehr als 28 cN/tex erhalten wurde.

Es sei darauf hingewiesen, dass die Ausführung der Erfindung nicht auf die in den Figuren beschriebene Ausführungsbeispiele beschränkt ist, sondern auch in einer Vielzahl von Varianten möglich ist.

## Patentansprüche

1. Bioresorbierbare und thermoplastisch verformbare Polymere mit oder ohne Formgedächtnis-Charakteristik, die in den Wiederholungseinheiten der Polymerketten röntgenopake Baugruppen enthalten und/oder mit bioresorbierbaren, röntgenopaken Nanopartikeln modifiziert sind.

2. Polymere nach Anspruch 1, wobei die genannten im Röntgenlicht sichtbaren, bioresorbierbaren und thermoplastisch verformbaren Polymeren Triiodphenyl-Seitengruppen als die genannten röntgenopake Baugruppen entsprechend der Formel (I) enthalten: 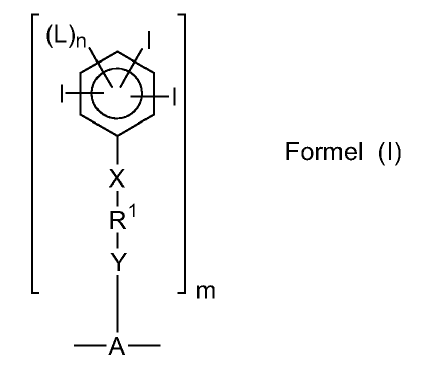 , wobei
L H oder eine wasserlöslichkeitsvermittelnde Carboxylat-, Ammonium-, Phosphat-, Phosphonat-, Sulfat- oder Sulfonat-Gruppe oder ein Oligoethylenoxid- oder Acetylamino-Rest darstellt,
n zwischen 0, 1 oder 2 variieren kann,
Y, X entfällt oder Verbindungsgruppen, wie Ether-, Carbonsäureester- oder Carbonsäureamid- oder Urethan-Gruppen darstellen,
R¹ entfällt oder ein 2-wertiger linearer, verzweigter oder cyclischer organischer Rest ist, der aus 1 bis 15 Kohlenstoffatomen besteht,
A ein m+2-wertiger linearer, verzweigter oder cyclischer organischer Rest ist, der 1 bis 30 Kohlenstoffatome enthält und
m zwischen 1 und 4 variieren kann und die Substitution des Phenylrestes durch die Iod-Atome in freie o-, m- oder p-Positionen erfolgt.

3. Polymere nach Anspruch 1, wobei die genannten im Röntgenlicht sichtbaren, bioresorbierbaren und thermoplastisch verformbaren Polymeren Triiodphenyl-Seitengruppen als die genannten röntgenopake Baugruppen entsprechend der Formel (I) enthalten: 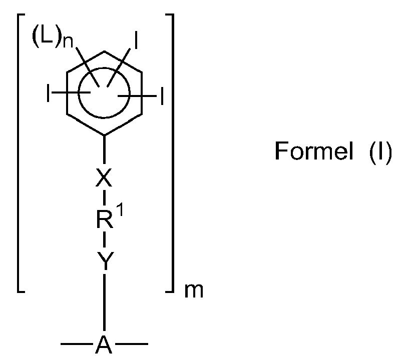 , wobei
L H, eine wasserlöslichkeitsvermittelnde Carboxylat-, Ammonium-, Phosphat-, Sulfat-Gruppe oder ein Oligoethylenoxid- oder Acetylamino-Rest darstellt,
n zwischen 0, 1 oder 2 variiert,
Y, X entfällt oder Verbindungsgruppen, wie Carbonsäureester- oder Urethan-Gruppen darstellen,
R¹ entfällt oder ein 2-wertiger linearer, verzweigter oder cyclischer organischer Rest ist, der aus 1 bis 10 Kohlenstoffatomen besteht,
A ein m+2-wertiger linearer, verzweigter oder cyclischer organischer Rest ist, der 1 bis 20 Kohlenstoffatome enthält und
m zwischen 1 und 2 variiert und
die Substitution des Phenylrestes durch die Iod-Atome in freie o- m- oder p-Positionen erfolgt.

4. Bioresorbierbare und thermoplastisch verformbare Polymere nach Anspruch 1, wobei die röntgenopaken Nanopartikel Nanokapseln darstellen, die aus einem Kern von röntgenopaken Triiodphenylverbindungen und einer Hülle aus bioresorbierbaren Polymeren aufgebaut sind.

5. Bioresorbierbare und thermoplastisch verformbare Polymere nach einem der vorangehenden Ansprüche, wobei die röntgenopaken Baugruppen in den bioresorbierbaren Polymeren durch Copolymerisation, Cokondensation bzw. Polyaddition eingebaut sind.

6. Bioresorbierbare und thermoplastisch verformbare Polymere nach Anspruch 5, wobei es sich bei den bioresorbierbaren Polymeren um Polyester, Polyanhydride, Polycarbonate, Polyamide oder Polyaminosäuren handelt.

7. Bioresorbierbare und thermoplastisch verformbare Polymere nach Anspruch 6, wobei es sich bei den Polyestern um Poly(α-hydroxycarbonsäuren) oder deren Copolymeren handelt.

8. Bioresorbierbare und thermoplastisch verformbare Polymere nach einem der vorangehenden Ansprüche, wobei die nach Einbau der röntgenopaken Baugruppen in den bioresorbierbaren Polymeren entstandenen unvernetzten röntgenopaken Polymere durch Diisocyanate vernetzt sind.

9. Bioresorbierbare und thermoplastisch verformbare Polymere nach einem der vorangehenden Ansprüche, wobei die nach Einbau der röntgenopaken Baugruppen in den bioresorbierbaren Polymeren entstandenen unvernetzten röntgenopaken Polymere durch Endgruppenmodifizierung mit radikalisch polymerisierbaren Gruppen versehen sind und die gebildeten Telechele anschließend in Gegenwart eines radikalischen Initiators und gegebenenfalls eines oder mehrerer radikalisch polymerisierbarer Comonomere unter Bildung eines bioabbaubaren, röntgenopaken Polymernetzwerks copolymerisiert sind.

10. Bioresorbierbare und thermoplastisch verformbare Polymere nach einem der vorangehenden Ansprüche, wobei den bioresorbierbaren und thermoplastisch verformbaren Polymeren vor oder während der Formgebung Additive zugesetzt sind, die eine Einstellung und Anpassung der mechanischen thermischen Eigenschaften und/oder spezifischen Applikationseigenschaften der Polymeren bewirken.

11. Verfahren zur Herstellung von bioresorbierbaren und thermoplastisch verformbaren Polymeren mit oder ohne Formgedächtnis-Charakteristik nach einem der Ansprüche 1-13, umfassend einbringen von röntgenopake Baugruppen in den Wiederholungseinheiten der Polymerketten und/oder modifizieren der Polymere mit bioresorbierbaren, röntgenopaken Nanopartikeln.

12. Verfahren gemäß Anspruch 11, zur Herstellung von bioresorbierbaren und thermoplastisch verformbaren Polymeren nach Anspruch 4, wobei die röntgenopaken Nanopartikel Nanokapseln darstellen, die aus einem Kern von röntgenopaken Triiodphenylverbindungen und einer Hülle aus bioresorbierbaren Polymeren aufgebaut sind, das Verfahren umfassend einsetzen von nichwasserlöslichen, bioabbaubaren Polyestern auf der Basis von α-Hydroxycarbonsäuren, deren Copolymere, Polyanhydride, Poly(α-aminosäure)n, Polyorthoester und/oder umfassend einsetzen von Polyphosphazene als Hüllmaterialien zur Herstellung der röntgenopaken Nanokapseln.

13. Verfahren gemäß Anspruch 11, zur Herstellung von bioresorbierbaren und thermoplastisch verformbaren Polymeren nach den Ansprüchen 1, 2 oder 3, umfassend einsetzen von multifunktionellen organischen Verbindungen zur Herstellung der röntgenopaken Baugruppen, wobei die multifunktionellen organischen Verbindungen über mindestens drei gleiche oder unterschiedliche funktionelle Gruppen verfügen.

14. Verfahren gemäß Anspruch 13, wobei zur Herstellung der röntgenopaken Baugruppen hydroxylgruppenhaltige Verbindungen mit mehr als drei Hydroxylgruppen pro Molekül eingesetzt werden, und wobei diese hydroxylgruppenhaltigen Verbindungen weitere funktionelle Gruppen enthalten können.

15. Verfahren gemäß Anspruch 11, umfassend einbauen der röntgenopaken Baugruppen in den bioresorbierbaren Polymeren durch Copolymerisation, Cokondensation bzw. Polyaddition.

16. Verfahren gemäß Anspruch einem der Ansprüche 11-15, wobei nach Einbau der röntgenopaken Baugruppen in den bioresorbierbaren Polymeren entstandene unvernetzte röntgenopaken Polymere durch Diisocyanate vernetzt werden.

17. Verfahren gemäß Anspruch einem der Ansprüche 11-16, wobei nach einem der vorangehenden Ansprüche, wobei die nach Einbau der röntgenopaken Baugruppen in den bioresorbierbaren Polymeren entstandenen unvernetzten röntgenopaken Polymere durch Endgruppenmodifizierung mit radikalisch polymerisierbaren Gruppen versehen werden und die gebildeten Telechele anschließend in Gegenwart eines radikalischen Initiators und gegebenenfalls eines oder mehrerer radikalisch polymerisierbarer Comonomere unter Bildung eines bioabbaubaren, röntgenopaken Polymernetzwerks copolymerisiert werden.

18. Verfahren gemäß Anspruch einem der Ansprüche 11-17, wobei den bioresorbierbaren und thermoplastisch verformbaren Polymeren vor oder während der Formgebung Additive zugesetzt werden, die eine Einstellung und Anpassung der mechanischen thermischen Eigenschaften und/oder spezifischen Applikationseigenschaften der Polymeren bewirken.

19. Verfahren gemäß Anspruch 11, umfassend umsetzen von Pentaerythrit (**PE**) in einer 1. Stufe durch Ketalisierung mit Dimethoxyaceton (**DMA**) zu einem Monoaceton-Pentaerythrit (**MAPE**), anschließend verestern der beiden freien OH-Gruppen in einer 2. Stufe mit 2,3,5-Triiodbenzoesäure **(asTIBA),** und in einer 3. Stufe die Aceton-Schutzgruppe sauer hydrolytisch wieder abgespalten, wodurch sich ein zwei Triiodphenyl-Seitengruppen enthaltendes Diol **PE-D(asTIBA)** bildet. Dies ist in Fig. 3 illustriert.

20. Verfahren gemäß Anspruch 19, alternativ umfassend verestern von Monoaceton-Pentaerythrit (**MAPE**) zum Einbau eines Spacers mit Glutarsäureanhydrid (**GA**), dann Einbau der beiden Triiodphenyl-Seitengruppen durch Umsetzung mit 2,4,6-Triiodphenol (**TIPh**), und schließlich abspalten der Aceton-Schutzgruppe in der letzten Stufe, wodurch sich das wiederum zwei Triiodphenyl-Seitengruppen enthaltene Diol **PE-GA-D(TIPh)** bildet.

21. Monofile oder Multifilamentgarne aus nach den Ansprüchen 1-4 hergestellten bioresorbierbaren und thermoplastisch verformbaren Polymeren, welche thermoplastisch oder nicht schmelzbar, aber löslich und aus der Schmelze oder Lösung heraus verarbeitbar sind.

22. Verfahren zur Herstellung von Monofilen oder Multifilamentgarnen gemäß Anspruch 21, wobei die Monofile oder Multifilamentgarne aus nach den Ansprüchen 1-4 bioresorbierbaren und thermoplastisch verformbaren Polymeren unter Nutzung einer Extrusionsspinnapparatur hergestellt werden.

23. Verfahren zur Herstellung von Monofilen oder Multifilamentgarnen gemäß Anspruch 21, wobei die Monofile oder Multifilamentgarne aus nach den Ansprüchen 1-4 bioresorbierbaren und thermoplastisch verformbaren Polymeren unter Nutzung einer Kolbenspinnapparatur hergestellt werden.

24. Verfahren zur Herstellung von Monofilen oder Multifilamentgarnen gemäß Anspruch 21, wobei die Monofile oder Multifilamentgarne aus nach den Ansprüchen 1-4 bioresorbierbaren und thermoplastisch verformbaren Polymeren unter Nutzung einer Spinnapparatur, insbesondere durch Lösungsspinnapparaturen, hergestellt werden.

25. Verfahren zur Herstellung von Monofilen oder Multifilamentgarnen gemäß Anspruch 21, wobei die Monofile oder Multifilamentgarne aus nach den Ansprüchen 1-4 bioresorbierbaren und thermoplastisch verformbaren Polymeren durch einen nach dem Spinnprozess stattfindenden Reckprozess verfestigt werden.

26. Verfahren zur Herstellung von Monofilen oder Multifilamentgarnen gemäß Anspruch 25, wobei der Spinn- und Reckprozess als Teilprozesse simultan in einem Verfahrensschritt vereint sind, oder als sequentiell selbstständige Prozesse räumlich und/oder zeitlich voneinander getrennt durchgeführt werden.

27. Verfahren zur Herstellung von Monofilen oder Multifilamentgarnen gemäß Anspruch 21, wobei die Monofile oder Multifilamentgarne aus nach den Ansprüchen 1-4 bioresorbierbaren und thermoplastisch verformbaren Polymeren, umfassend verfestigen der Monofile oder Multifilamentgarne durch Tempern, wobei Wärme durch heiße Gase oder Gemische von Gasen, heiße Flüssigkeiten oder durch Strahlung übertragen wird.

28. Verfahren zur Herstellung von Monofilen oder Multifilamentgarnen gemäß Anspruch 24, wobei die Monofile oder Multifilamentgarne durch Vernetzung verfestigt werden, wobei die Vernetzung durch Strahlung insbesonders durch γ- oder UV-Strahlung initiiert wird.

29. Verfahren zur Herstellung von Multifilamentgarnen gemäß Anspruch 22, wobei der Zusammenhalt des Fadenverbands des Multifilamentgarnes durch Aufbringen von Drehungen stabilisiert und verbessert wird.

30. Verfahren zur Herstellung von Multifilamentgarnen gemäß Anspruch 22, wobei der Zusammenhalt des Fadenverbands des Multifilamentgarnes durch Verwirbeln und Bildung von Fadenverschlingungen stabilisiert und verbessert wird.

31. Verfahren zur Herstellung von Multifilamentgarnen gemäß Anspruch 22, wobei der Zusammenhalt des Fadenverbands des Multifilamentgarnes durch Aufbringen adhäsiv wirkender Substanzen, wie Schlichte, Präparation oder Finish, stabilisiert und verbessert wird.

32. Occlussionsinstrument zum Verschluss von Defekten des Septums im Herzen umfassend ein bioresorbierbare und thermoplastisch verformbare Polymeren nach einem der Ansprüche 1-10.

33. Occlussionsinstrument gemäß Anspruch 32, hergestellt nach einem Verfahren gemäß einem der Ansprüche 22-31.

34. Verfahren zur Herstellung von Occlusionsinstrumenten nach Anspruch 32, wobei durch Lösungs- oder Schmelzspinnen aus den bioresorbierbaren und thermoplastisch verformbaren Polymeren mit oder ohne Formgedächtnis-Charakteristik, die einerseits in den Wiederholungseinheiten der Polymerketten röntgenopake Triiodphenyl-Seitengruppen entsprechend der Formel (I) enthalten und/oder andererseits mit bioresorbierbaren, röntgenopaken Nanopartikeln modifiziert sind, Fäden hergestellt werden, die nachfolgend durch Formgebungsverfahren zu Occlusionsinstrumenten verarbeitet werden.

35. Verwendung von bioresorbierbaren und thermoplastisch verformbaren Polymeren nach den Ansprüchen 1-4 für die Herstellung von kollabierbaren Occlusionsinstrumenten, chirurgischen Formkörpern oder Implantaten.

36. Verwendung von bioresorbierbaren und thermoplastisch verformbaren Polymeren nach den Ansprüchen 1-4 für die Herstellung von kollabierbaren Occlusionsinstrumenten, die zum Verschluss von Defekten des Septums im Herzen dienen, wobei deren Platzierung mit röntgendiagnostischen Verfahren verfolgbar ist.

37. Verwendung von bioresorbierbaren und thermoplastisch verformbaren Polymeren nach Anspruch 4 für die Herstellung von Occlusionsinstrumenten, chirurgischen Formkörpern oder Implantaten, wobei für die Herstellung der röntgenopaken Nanokapseln wasserlösliche bioresorbierbare Polysaccharide, Polysaccharidderivate, Proteine oder Polyvinylalkohol als Hüllmaterialien eingesetzt werden.
